(19) Europäisches Patentamt
European Patent Office
Office européen des brevets

(11) **EP 4 810 528 A1**

(12) **EUROPEAN PATENT APPLICATION**

(43) Date of publication:
**23.09.2026 Bulletin 2026/39**

(21) Application number: **26165249.9**

(22) Date of filing: **16.03.2026**

(51) International Patent Classification (IPC):
***C08B 37/00*** *(2006.01)* ***C08B 37/08*** *(2006.01)*

(52) Cooperative Patent Classification (CPC):
**C08B 37/0003; C08B 37/003**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **17.03.2025 PL 45148525**

(71) Applicant: **Akademia Gorniczo-Hutnicza im. Stanislawa Staszica w Krakowie
30-059 Krakow (PL)**

(72) Inventors:
- **Swistun, Eugeniusz
47-300 Krapkowice (PL)**
- **Zdziobek, Patryk
31-422 Kraków (PL)**

(74) Representative: **Galazkiewicz, Wlodzimierz
THINKPAT Sp. z o.o.
ul. Iglasta 8
05-825 Radonie (PL)**

Remarks:
Amended claims in accordance with Rule 137(2) EPC.

# (54) METHOD FOR PRODUCING CHITOSAN FROM FOREST WASTE

(57) The invention provides a method for producing chitosan from a waste material in the form of forest insects, such as forest pests. The method comprises a sequence of optimized chemical steps, including drying dead insects, their homogenization into a powdered form, and subsequently demineralization in a hydrochloric acid solution, deproteinization in a sodium hydroxide solution, and deacetylation in a concentrated sodium hydroxide solution at an elevated temperature. The specific combination of process parameters allows for the effective processing of forest waste into high-quality chitosan with a surprisingly high yield.

chitin
chitosan
efficiency of material production in percent of dry mass
20
16
12
8
4
0
Omocestus viridulus
Crangon crangon
Ips typographus
Tenebrio molitor
Gryllus assimilis

FIG. 2

EP 4 810 528 A1

## Description

### FIELD OF THE INVENTION

The present invention relates to a method for producing chitosan, in particular from a waste material in the form of forest insects.

### BACKGROUND OF THE INVENTION

Chitosan is a natural biopolymer with a broad spectrum of applications in medicine, agriculture, cosmetology, and environmental protection. Traditionally, the main source of chitosan is the exoskeletons of marine crustaceans, such as shrimp and crabs. As disclosed, for example, in patent application US 2016/0237176 A1, the production process from crustaceans includes the steps of demineralization, deproteinization, and deacetylation. However, sourcing the raw material from these sources is associated with a number of limitations, such as seasonality, dependence on imports, high transportation costs, and a negative impact on marine ecosystems. The prior art describes attempts to use insects as an alternative source of chitin and chitosan. For example, comprehensive review articles, such as the publication by Mohan et al. entitled "Recent insights into the extraction, characterization, and bioactivities of chitin and chitosan from insects" (Trends in Food Science & Technology, 2020, 105, 17-42) and the publication by Hahn et al. entitled "Current state of chitin purification and chitosan production from insects" (Journal of Chemical Technology and Biotechnology, 2020, 95, 2775-2795), describe the general concept of chemical extraction of chitosan from various insect species.

Furthermore, scientific publications, such as "Insect residues as an alternative and promising source for the extraction of chitin and chitosan" (International Journal of Biological Macromolecules, 2024, 254) and "Insect-Derived Chitin and Chitosan: A Still Unexploited Resource for the Edible Insect Sector" (Sustainability, 2023), confirm that insects are considered a sustainable alternative.

Other documents describe specific method variants, which, however, differ from the method according to the invention. Chinese patent application CN 106243245 A discloses a method in which the deproteinization step is conducted enzymatically, not chemically, and the deacetylation process is assisted by ultrasound and UV radiation. In turn, Russian patent RU 2615636 C1 describes a process for producing chitosan from the pupae of the housefly (*Musca domestica*). The state of the art, however, does not provide motivation to investigate difficult and non-uniform raw materials such as forest waste. During gradations (mass appearances of insects), insects such as the European spruce bark beetle, the common cockchafer, the May beetle, the common pine weevil, or the nun moth are trapped in large quantities. The captured insects have no application and are stored at the collection site, where they undergo biodegradation involving microorganisms and other organisms present in the soil. As forest waste, they constitute a difficult and non-uniform raw material.

In light of the known prior art, there is therefore a need to develop an efficient and economically viable method for the valorization of a specific type of waste, namely insects that are forest pests, in order to obtain high-quality chitosan.

### SUMMARY OF THE INVENTION

The object of the present invention is to solve the above problems by providing a method for producing chitosan that utilizes forest waste in the form of insects as a raw material. This method, thanks to a specific combination of process parameters, allows for the production of chitosan with high purity and a surprisingly high yield.

### BRIEF DESCRIPTION OF THE DRAWINGS

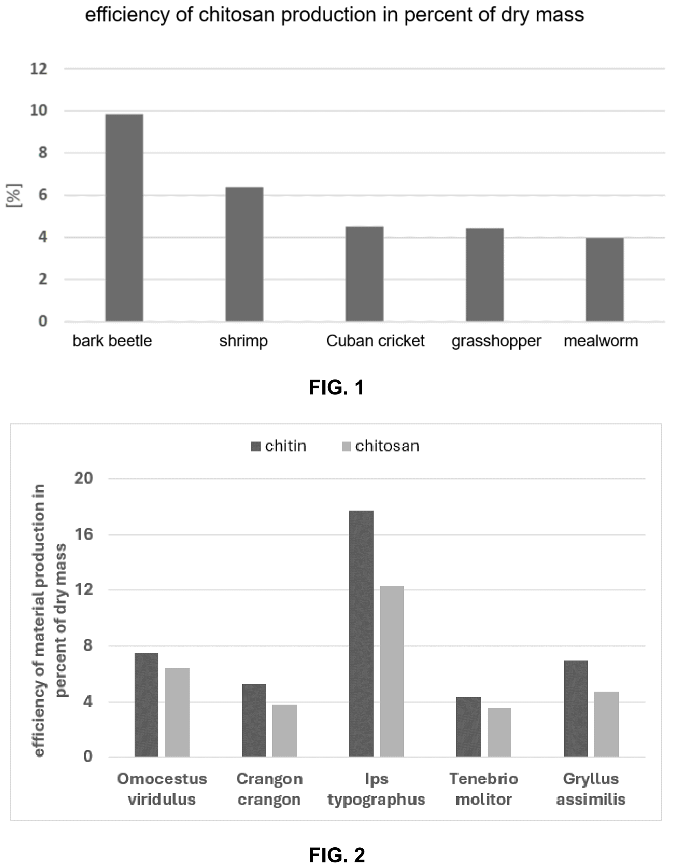

The subject matter of the invention is further explained in the embodiments and in the drawings, in which: **FIG. 1** presents a bar chart showing the efficiency of chitosan production in percent of dry mass for various sources. **FIG. 2** graphically presents the efficiency results from Example 4.

### DETAILED DESCRIPTION OF THE INVENTION

The present invention provides an optimized method for producing chitosan from a waste material in the form of forest insects, such as pests massively trapped in forest traps, e.g., the European spruce bark beetle (*Ips typographus*) or the common cockchafer (*Melolontha melolontha*). The present invention provides a method for producing chitosan from a waste material in the form of forest insects, such as pests massively trapped in forest traps. This method, in its general aspect, comprises a sequence of chemical steps aimed at isolating and converting chitin to chitosan.

In a first step (a), dead insects, constituting the starting material, are subjected to drying. This step aims to remove water from the biomass, which is crucial for the efficiency of further chemical reactions. The process is conducted at a temperature in the range of 30 °C to 121 °C to achieve a moisture content in the range of 0% to 5%. In a preferred

embodiment, the drying is carried out at a temperature from 55 °C to 105 °C. In the most advantageous embodiment, the drying temperature is 60 °C.

Next, in step (b), the dried material is subjected to homogenization, for example by grinding, to obtain a powdered form. This increases the specific surface area of the material and facilitates the penetration of reagents. In an advantageous embodiment, homogenization is carried out to obtain a powder with an average particle size in the range of 200-1500 μm, preferably 200-500 μm.

In step (c), the powdered material is subjected to demineralization. This process involves removing mineral salts from the insect exoskeletons using an acid solution. The process is carried out in a hydrochloric acid (HCl) solution with a concentration in the range of 0.2 M to 3 M, at a temperature from 10 °C to 75 °C, for a duration of 0.25 h to 72 h, at a mass-to-volume ratio from 1:5 to 1:40 (g/ml). In a preferred embodiment, demineralization is carried out in an HCl solution with a concentration in the range of 1 M to 2 M, at a temperature from 20 °C to 30 °C, for a duration of 24 h. In the most advantageous embodiment, the process is carried out in an HCl solution with a concentration of 1 M, at a mass-to-volume ratio of 1:10 (g/ml), at a temperature of 20 °C, for a duration of 12 hours.

The next step (d), deproteinization, aims to remove proteins from the material. It is carried out using a base solution. The process is conducted in a sodium hydroxide (NaOH) solution with a concentration from 0.2 M to 19 M, at a mass-to-volume ratio from 1:5 to 1:40 (g/ml), at a temperature from 30 °C to 100 °C, for a duration from 0.25 h to 72 h. In a preferred embodiment, deproteinization is carried out in an NaOH solution with a concentration from 1 M to 2 M, at a temperature of 70 °C, for a duration from 2 h to 16 h. In the most advantageous embodiment, the process is carried out in an NaOH solution with a concentration of 1 M, at a mass-to-volume ratio of 1:10 (g/ml), at a temperature of 70 °C, for a duration of 14 hours.

The key step is deacetylation (e), during which chitin is converted into chitosan by removing acetyl groups. The process is carried out in a concentrated base solution at an elevated temperature. The process is conducted in an NaOH solution with a concentration from 8 M to 19 M, at a mass-to-volume ratio from 1:5 to 1:40 (g/ml), at a temperature from 60 °C to 130 °C, for a duration from 1/6 h to 20 h. In a preferred embodiment, deacetylation is carried out in an NaOH solution with a concentration from 13 M to 16 M, at a mass-to-volume ratio from 1:5 to 1:40 (g/ml), at a temperature from 100 °C to 120 °C, for a duration from 1 h to 3 h. In the most advantageous embodiment, the process is carried out in an NaOH solution with a concentration of 13 M, at a mass-to-volume ratio of 1:10 (g/ml), at a temperature in the range of 100 °C to 120 °C, for a duration of 2 hours.

Finally, in step (f), the purified product is dried at a temperature in the range of 30 °C to 121 °C. After any of the steps selected from demineralization, deproteinization, and deacetylation, the material is preferably washed with distilled water until a neutral pH is achieved.

In certain embodiments of the invention, to intensify the process, during the demineralization (c), deproteinization (d), or deacetylation (e) steps, the material may be subjected to a process of mechanical mixing (1-700 rpm, preferably 150 rpm), sonification (ultrasound irradiation for up to 4 hours, preferably 1 h), or microwave irradiation (up to 600 W, preferably 400 W, for a duration of 5 minutes to 1 h).

In another embodiment, to improve product quality and reaction efficiency, the deacetylation step (e) may be carried out under vacuum or in an inert gas atmosphere, such as nitrogen or argon. Preferably, the deacetylation process is carried out under vacuum using an autoclave. To assist the process and the condensation of NaOH vapors, a vapor condensation system can be used. The obtained chitosan may optionally be subjected to an additional step of grinding or decolorization. Decolorization can be carried out using oxidizing agents, such as $H_2O_2$ solution, or organic solvents, such as ethanol or acetone.

In an advantageous embodiment, the forest insects are dead arthropods from the Scarabaeidae, Curculionidae, or Lymantriidae family. Exemplary arthropods from the Scarabaeidae family are the common cockchafer, the May beetle, the summer chafer; from the Curculionidae family: the European spruce bark beetle, the six-toothed bark beetle, the common pine weevil; from the Lymantriidae family: the nun moth.

**Example 1**

The production of chitosan from insects, using the example of the common cockchafer obtained from a forest trap, involved the following steps:

**a) Preliminary drying**
Dead insects (common cockchafer) with a mass of 42.24 g were dried in a laboratory dryer to dryness at 105 °C.

**b) Homogenization**
The dried insects were ground into a powder with a particle size of 200-1500 μm using a laboratory mill for about 3 minutes.

**c) Demineralization**
16.46 g of the waste material was mixed with 164.5 ml of 1M HCl and thoroughly stirred. The material was left for 24 h,

then the obtained material after demineralization was rinsed with distilled water until a neutral pH value was achieved.

**d) Deproteinization**

11.71 g of the waste material was mixed with 117.7 ml of 1 M NaOH and heated with stirring at 70 °C for 14 h, then rinsed with distilled water until a neutral pH of 7 was achieved.

**e) Deacetylation**

2.94 g of the waste material was mixed with 29.4 ml of 13 M NaOH and heated at 120 °C for 2h under vacuum. The material obtained after deacetylation was rinsed with distilled water until a neutral pH value was achieved.

**f) Final drying**

Finally, the finished chitosan from insects was dried in a laboratory dryer at 105 °C for 24 h, ground, and decolorized with an $H_2O_2$ solution for 10 min.

**Example 2**

The production of chitosan from insects, using the example of the European spruce bark beetle obtained from a forest trap, involved the following steps:

**a) Preliminary drying**

Dead insects (European spruce bark beetle) with a mass of 145.45 g were dried in a laboratory dryer to dryness at 105 °C.

**b) Homogenization**

The dried insects were ground into a powder with a particle size of 200-1500 μm using a laboratory mill for about 3 minutes, then using a mortar for about 3 minutes to obtain a particle size in the range of 200-500 μm.

**c) Demineralization**

56.68 g of the waste material was mixed with 1700 ml of 2M HCl and stirred with a magnetic stirrer (150 rpm) at 30 °C for 24 h. The material obtained after demineralization was then rinsed with distilled water until a neutral pH value was achieved.

**d) Deproteinization**

35.20 g of the waste material was mixed with 1056 ml of 2 M NaOH and subjected to sonification for 2 h, then rinsed with distilled water until a neutral pH of 7 was achieved.

**e) Deacetylation**

14.60 g of the waste material was mixed with 438 ml of 16 M NaOH and heated at 100°C for 3 h, stirred with a magnetic stirrer (650 rpm), and an NaOH vapor condensation system was used. The material obtained after deacetylation was rinsed with distilled water until a neutral pH value was achieved.

**f) Final drying and processing**

Finally, the finished chitosan from insects was dried in a laboratory dryer at 105°C for 24 h, ground, and decolorized with an $H_2O_2$ solution.

**Example 3**

The process was carried out analogously to Example 2, with the difference that in the preliminary drying step (a), a temperature of 60 °C was used.

**Example 4**

The production of chitosan from various arthropods, including the European spruce bark beetle obtained from a forest trap, involved the following steps:

**a) Preliminary drying**

Dead arthropods, i.e., adult European spruce bark beetle (*Ips typographus*), adult field grasshopper (*Omocestus viridulus*), mealworm larvae (*Tenebrio molitor*), adult Cuban cricket (*Gryllus assimilis*), adult shrimp (*Crangon crangon*), were dried in a laboratory dryer at 55 °C to dryness.

**b) Homogenization**

The dried arthropods were ground into a fine powder (with an average particle size of 200-1500 μm) using a laboratory mill for about 3 minutes. In subsequent steps, chitin extraction and chitosan production were carried out. The masses of the individual arthropods for the subsequent steps are given in Table 1.

**c) Demineralization**

The indicated amount of material was mixed with 1M HCl in a quantity of 30 ml/g and stirred with a magnetic stirrer (150 rpm) at 30 °C for 24 h. The obtained material was then rinsed with distilled water until a neutral pH value was achieved

and dried for 24 h at 55 °C.

**d) Deproteinization**

The indicated amount of material was mixed with 1M NaOH in a quantity of 30 ml/g and stirred with a magnetic stirrer (150 rpm) at 70 °C for 16 h. The obtained material was then rinsed with distilled water until a neutral pH value was achieved and dried for 24 h at 55 °C.

**e) Deacetylation**

The indicated amount of material was mixed with 13M NaOH in a quantity of 30 ml/g and heated at 120°C for 1 h, stirred with a magnetic stirrer (650 rpm). The material obtained after deacetylation was rinsed with distilled water until a neutral pH value was achieved.

**f) Final drying**

The product was then dried for 24 h at 55 °C.

**Table 1**

| | Initial mass [g] | Amount of 1M HCl added [ml] | Mass after demineralization [g] | Amount of 1M NaOH added [ml] | Mass after deproteinization [g] | Amount of 13M NaOH added [ml] | Mass after deacetylation [g] | Chitin yield in % of dry mass | Chitosan yield in % of dry mass |
|---|---|---|---|---|---|---|---|---|---|
| bark beetle | 22.84 | 685.05 | 15.02 | 450.55 | 1.71 | 51.30 | 1.46 | 7.49 | 6.38 |
| shrimp | 28.85 | 865.59 | 11.16 | 334.78 | 1.52 | 45.60 | 1.09 | 5.27 | 3.78 |
| bark beetle | 12.62 | 378.45 | 8.46 | 253.81 | 2.24 | 67.20 | 1.55 | 17.76 | 12.31 |
| mealworm | 21.18 | 635.27 | 18.04 | 541.15 | 0.92 | 27.60 | 0.75 | 4.34 | 3.55 |
| cricket | 12.07 | 362.15 | 10.40 | 312.03 | 0.84 | 25.20 | 0.56 | 6.96 | 4.68 |

As shown in the studies and in **FIG. 2,** the method according to the invention, applied to forest waste in the form of the European spruce bark beetle, allowed for a chitosan yield of up to 12.31% of dry mass. This is an unexpectedly and advantageously higher result than the yield obtained from traditional sources, such as shrimp, or from insects that are not forest waste.

## Claims

**1.** A method for producing chitosan from a waste material in the form of forest insects, comprising the steps of homogenization, demineralization, deproteinization, deacetylation, washing, and drying, **characterized in that** it comprises the following steps:

a) drying dead insects at a temperature from 30 °C to 121 °C to obtain a moisture content in the range of 0 % to 5 %;
b) homogenizing the dried insects from step a) to obtain a powder;
c) demineralizing the powder obtained in step b) in a hydrochloric acid (HCl) solution with a concentration from 0.2 M to 3 M, at a mass-to-volume ratio from 1:5 to 1:40 (g/ml), for a duration from 0.25 h to 72 h, at a temperature from 10 °C to 75 °C;
d) deproteinizing the material obtained in step c) in a sodium hydroxide (NaOH) solution with a concentration from 0.2 M to 19 M, at a mass-to-volume ratio from 1:5 to 1:40 (g/ml), for a duration from 0.25 h to 72 h, at a temperature from 30 °C to 100 °C;
e) deacetylating the material obtained in step d) in an NaOH solution with a concentration from 8 M to 19 M, at a mass-to-volume ratio from 1:5 to 1:40 (g/ml), for a duration from 1/6 h to 20 h, at a temperature in the range of 60 °C to 130 °C; and
f) drying the washed material at a temperature in the range of 30°C to 121°C.

**2.** A method according to claim 1, **characterized in that** it comprises the following steps:

a) drying dead insects at a temperature from 55 °C to 105 °C to obtain a moisture content in the range of 0% to 5%;
b) homogenizing the dried insects from step a) to obtain a powder;
c) demineralizing the powder obtained in step b) in a hydrochloric acid (HCl) solution with a concentration from 1 M to 2 M, at a mass-to-volume ratio from 1:5 to 1:40 (g/ml), for 24 h, at a temperature from 20 °C to 30 °C;
d) deproteinizing the material obtained in step c) in a sodium hydroxide (NaOH) solution with a concentration from 1 M to 2 M, at a mass-to-volume ratio from 1:5 to 1:40 (g/ml), for a duration from 2 h to 16 h, at a temperature from 20 °C to 70 °C;
e) deacetylating the material obtained in step d) in an NaOH solution with a concentration from 13 M to 16 M, at a mass-to-volume ratio from 1:5 to 1:40 (g/ml), for a duration from 1 h to 3 h, at a temperature in the range of 100 °C to 120 °C; and
f) drying the washed material at a temperature in the range of 30°C to 121°C.

**3.** A method according to claim 1, **characterized in that** it comprises the following steps:

a) drying dead insects at a temperature of 60 °C to obtain a moisture content in the range of 0% to 5%;
b) homogenizing the dried insects from step a) to obtain a powder;
c) demineralizing the powder obtained in step b) in a hydrochloric acid (HCl) solution with a concentration of 1 M, at a mass-to-volume ratio of 1:10 (g/ml), for 12 h, at a temperature of 20 °C;
d) deproteinizing the material obtained in step c) in a sodium hydroxide (NaOH) solution with a concentration of 1 M, at a mass-to-volume ratio of 1:10 (g/ml), for 14 h, at a temperature of 70 °C;
e) deacetylating the material obtained in step d) in an NaOH solution with a concentration of 13 M, at a mass-to-volume ratio of 1:10 (g/ml), for 2 h, at a temperature in the range of 100 °C to 120 °C; and
f) drying the washed material at a temperature in the range of 30°C to 121°C.

**4.** A method according to any one of the preceding claims, **characterized in that** after any of the steps selected from c), d), and e) the material is washed with distilled water until a neutral pH is achieved.

**5.** A method according to any one of the preceding claims, **characterized in that** the forest insects are dead arthropods from the Scarabaeidae, Curculionidae, or Lymantriidae family.

**6.** A method according to any one of the preceding claims, **characterized in that** the homogenization in step b) is carried out to obtain a powder with an average particle size in the range of 200-500 μm.

**7.** A method according to any one of the preceding claims, **characterized in that** during the demineralization, deproteinization, or deacetylation steps, the material is subjected to a process of mixing, sonification, or microwave irradiation.

**8.** A method according to any one of the preceding claims, **characterized in that** the deacetylation step e) is carried out under vacuum or in an inert gas atmosphere.

**9.** A method according to any one of the preceding claims, **characterized in that** it additionally comprises a step of grinding or decolorizing the obtained chitosan, wherein the decolorization is carried out with an $H_2O_2$ solution, soluble chlorine compounds, ethanol, or acetone.

**Amended claims in accordance with Rule 137(2) EPC.**

**1.** A method for producing chitosan from a waste material in the form of forest insects, comprising the steps of homogenization, demineralization, deproteinization, deacetylation, washing, and drying, **characterized in that** the forest insects are dead arthropods from the Scarabaeidae, Curculionidae, or Lymantriidae family and it comprises the following steps:

a) drying dead insects at a temperature from 30°C to 121 °C to obtain a moisture content in the range of 0 % to 5 %;
b) homogenizing the dried insects from step a) to obtain a powder;
c) demineralizing the powder obtained in step b) in a hydrochloric acid (HCl) solution with a concentration from 0.2 M to 3 M, at a mass-to-volume ratio from 1:5 to 1:40 (g/ml), for a duration from 0.25 h to 72 h, at a temperature from 10 °C to 75 °C;
d) deproteinizing the material obtained in step c) in a sodium hydroxide (NaOH) solution with a concentration from 0.2 M to 19 M, at a mass-to-volume ratio from 1:5 to 1:40 (g/ml), for a duration from 0.25 h to 72 h, at a temperature

from 30 °C to 100 °C;
e) deacetylating the material obtained in step d) in an NaOH solution with a concentration from 8 M to 19 M, at a mass-to-volume ratio from 1:5 to 1:40 (g/ml), for a duration from 1/6 h to 20 h, at a temperature in the range of 60 °C to 130 °C; and
f) drying the washed material at a temperature in the range of 30°C to 121°C.

**2.** A method according to claim 1, **characterized in that** it comprises the following steps:

a) drying dead insects at a temperature from 55 °C to 105 °C to obtain a moisture content in the range of 0% to 5%;
b) homogenizing the dried insects from step a) to obtain a powder;
c) demineralizing the powder obtained in step b) in a hydrochloric acid (HCl) solution with a concentration from 1 M to 2 M, at a mass-to-volume ratio from 1:5 to 1:40 (g/ml), for 24 h, at a temperature from 20 °C to 30 °C;
d) deproteinizing the material obtained in step c) in a sodium hydroxide (NaOH) solution with a concentration from 1 M to 2 M, at a mass-to-volume ratio from 1:5 to 1:40 (g/ml), for a duration from 2 h to 16 h, at a temperature from 20 °C to 70 °C;
e) deacetylating the material obtained in step d) in an NaOH solution with a concentration from 13 M to 16 M, at a mass-to-volume ratio from 1:5 to 1:40 (g/ml), for a duration from 1 h to 3 h, at a temperature in the range of 100 °C to 120 °C; and
f) drying the washed material at a temperature in the range of 30°C to 121°C.

**3.** A method according to claim 1, **characterized in that** it comprises the following steps:

a) drying dead insects at a temperature of 60 °C to obtain a moisture content in the range of 0% to 5%;
b) homogenizing the dried insects from step a) to obtain a powder;
c) demineralizing the powder obtained in step b) in a hydrochloric acid (HCl) solution with a concentration of 1 M, at a mass-to-volume ratio of 1:10 (g/ml), for 12 h, at a temperature of 20 °C;
d) deproteinizing the material obtained in step c) in a sodium hydroxide (NaOH) solution with a concentration of 1 M, at a mass-to-volume ratio of 1:10 (g/ml), for 14 h, at a temperature of 70 °C;
e) deacetylating the material obtained in step d) in an NaOH solution with a concentration of 13 M, at a mass-to-volume ratio of 1:10 (g/ml), for 2 h, at a temperature in the range of 100 °C to 120 °C; and
f) drying the washed material at a temperature in the range of 30°C to 121°C.

**4.** A method according to any one of the preceding claims, **characterized in that** after any of the steps selected from c), d), and e) the material is washed with distilled water until a neutral pH is achieved.

**5.** A method according to any one of the preceding claims, **characterized in that** the homogenization in step b) is carried out to obtain a powder with an average particle size in the range of 200-500 μm.

**6.** A method according to any one of the preceding claims, **characterized in that** during the demineralization, deproteinization, or deacetylation steps, the material is subjected to a process of mixing, sonification, or microwave irradiation.

**7.** A method according to any one of the preceding claims, **characterized in that** the deacetylation step e) is carried out under vacuum or in an inert gas atmosphere.

**8.** A method according to any one of the preceding claims, **characterized in that** it additionally comprises a step of grinding or decolorizing the obtained chitosan, wherein the decolorization is carried out with an $H_2O_2$ solution, soluble chlorine compounds, ethanol, or acetone.

efficiency of chitosan production in percent of dry mass
12
10
8
6
4
2
0
[%]
bark beetle
shrimp
Cuban cricket
grasshopper
mealworm

**FIG. 1**

chitin
chitosan
20
16
12
8
4
0
efficiency of material production in percent of dry mass
Omocestus viridulus
Crangon crangon
Ips typographus
Tenebrio molitor
Gryllus assimilis

**FIG. 2**

Europäisches Patentamt
European Patent Office
Office européen des brevets

# EUROPEAN SEARCH REPORT

Application Number
EP 26 16 5249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MACHADO SINARA SILVA NEVES ET AL: "Insect residues as an alternative and promising source for the extraction of chitin and chitosan", INTERNATIONAL JOURNAL OF BIOLOGICAL MACROMOLECULES ELSEVIER BV, NL, vol. 254, 3 November 2023 (2023-11-03), XP087442631, ISSN: 0141-8130, DOI: 10.1016/J.IJBIOMAC.2023.127773 [retrieved on 2023-11-03] * abstract * * Sections 2., 2.2.1. and 2.2.2. * * figure 1; table 1 * ----- | 1-9 | INV. C08B37/00 C08B37/08 |
| X | KEERTHIKA KANNAN ET AL: "Extraction, characterization and evaluation of antimicrobial activity of chitosan from adult Zophobas morio (Fabricius, 1776) (Coleoptera: Tenebrionidae)", INTERNATIONAL JOURNAL OF BIOLOGICAL MACROMOLECULES ELSEVIER BV, NL, vol. 279, 30 August 2024 (2024-08-30), XP087625142, ISSN: 0141-8130, DOI: 10.1016/J.IJBIOMAC.2024.135188 [retrieved on 2024-08-30] * abstract * * 2.Materials and methods and 2.3. Chitosan extraction * ----- -/-- | 1-9 | TECHNICAL FIELDS SEARCHED (IPC) C08B |

The present search report has been drawn up for all claims

2

| Place of search | Date of completion of the search | Examiner |
|---|---|---|
| Munich | 14 July 2026 | Lartigue, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number

EP 26 16 5249

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HAHN THOMAS ET AL: "Current state of chitin purification and chitosan production from insects", JOURNAL OF CHEMICAL TECHNOLOGY AND BIOTECHNOLOGY, vol. 95, no. 11, 25 August 2020 (2020-08-25), pages 2775-2795, XP093385230, Hoboken, USA ISSN: 0268-2575, DOI: 10.1002/jctb.6533 * Left-hand column, first whole paragraph; page 2781 * ----- | 1-9 | |

TECHNICAL FIELDS SEARCHED (IPC)

The present search report has been drawn up for all claims

| Place of search | Date of completion of the search | Examiner |
|---|---|---|
| Munich | 14 July 2026 | Lartigue, M |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20160237176 A1
- CN 106243245 A
- RU 2615636 C1


### Non-patent literature cited in the description


- **MOHAN et al.** Recent insights into the extraction, characterization, and bioactivities of chitin and chitosan from insects. *Trends in Food Science & Technology*, 2020, vol. 105, 17-42
- **HAHN et al.** Current state of chitin purification and chitosan production from insects. *Journal of Chemical Technology and Biotechnology*, 2020, vol. 95, 2775-2795
- Insect residues as an alternative and promising source for the extraction of chitin and chitosan. *International Journal of Biological Macromolecules*, 2024, vol. 254
- Insect-Derived Chitin and Chitosan: A Still Unexploited Resource for the Edible Insect Sector. *Sustainability*, 2023